# EUROPÄISCHE PATENTSCHRIFT

(11) **EP 0 551 632 B1**
(45) Veröffentlichungstag und Bekanntmachung des Hinweises auf die Patenterteilung: **26.06.1996**
(21) Anmeldenummer: 92121658.6
(22) Anmeldetag: 19.12.1992
(51) Int. Cl.: C07C 227/08, C07C 229/56, C07C 25/02, C07C 25/13, C07C 63/70

(54) **Verfahren zur Herstellung von Halogenanthranilsäuren**
Process for the preparation of halogenoanthranilic acid
Procédé de préparation d'acide anthranilique halogénée

(30) Priorität: 11.01.1992 DE 4200512
(43) Veröffentlichungstag der Anmeldung: 21.07.1993
(73) Patentinhaber: RIEDEL-DE HAEN AKTIENGESELLSCHAFT, D-30926 Seelze (DE)
(72) Erfinder: Schmand, Horst, Dr., W-3052 Bad Nenndorf (DE); Kellermeier, Bernd, W-3067 Lindhorst (DE); Bartels, Günter, Dr., W-3006 Burgwedel (DE); Schmidt, Hans-Jürgen, Dr., W-3016 Seelze (DE)
(74) Vertreter: Geissler, Bernhard, Dr. jur., Dipl.-Phys.

(56) Entgegenhaltungen:
- EP-A- 0 370 686
- DE-C- 244 207
- COMPTES RENDUES Bd. 202, Nr. 14, 6. April 1936, PARIS (FR) Seiten 1795 - 1796 R. PAJEAU 'The action of excess bromine on several derivatives of benzene in the presence of beryllium bromide'
- DATABASE WPI Week 9304, Derwent Publications Ltd., London, GB; AN 93-030505 6 JP-A-4 356 449 (KI KASEI KK)
- Chemical Abstracts, Vol 30, Abst. no. 5950-8, 1936, "Action of amines upon esters".
- Beilsteins Handbuch der Organischen Chemie, Formelregister, Band 5, Seite 36, "C7H5Br2Cl".
- Beilsteins Handbuch der Organischen Chemie, Drittes Ergänzungswerk, Seite 718, E III 5.
- Beilsteins Handbuch der organischen Chemie, Drittes Ergänzungswerk, Seite 1429, E III 9.
- Beilsteins Handbuch der Organischen Chemie, Gesamtregister für das Haptwerk und Ergänzungswerke I,II,III und IV, Seite 28, Band 9-11.
- Encyclopedia of Chemical Technology, 3rd Edition, Volume 2, page 474

## Beschreibung

Die vorliegende Erfindung betrifft ein Verfahren zur Herstellung von Halogenanthranilsäuren, sowie neue Zwischenprodukte zur Herstellung von Halogenanthranilsäuren.

Halogenanthranilsäuren sind wertvolle Zwischenprodukte zur Herstellung von Pharmazeutika (siehe beispielsweise US 4,833,270) bzw. Pflanzenschutzmitteln (siehe beispielsweise EP-A 360 417).

Verfahren zu ihrer Herstellung sind bereits bekannt. So ist beispielsweise in der DE-A 34 09 244 die Oxidation von Halogen-2-nitrotoluol mit 30 %iger wäßriger Salpetersäure bei erhöhter Temperatur und erhöhtem Druck zur entsprechenden Benzoesäure und anschließende Reduktion der Nitrogruppe beschrieben. Aufgrund der Verwendung von 30 %iger wäßriger Salpetersäure sind allerdings für eine industrielle Nutzung dieses Verfahrens Reaktionsbehälter aus speziellen und damit sehr teuren Werkstoffen erforderlich.

Die EP-A 342 849 beschreibt den Aufbau von Halogenisatinen aus Halogenanilinen und anschließender oxidativer alkalischer Ringöffnung zu den Halogenanthranilsäuren. Nachteilig an diesem Verfahren ist, daß alle Schritte mit erheblichen Ausbeuteverlusten verbunden sind. Beispielsweise beträgt die Gesamtausbeute an 4,5-Difluoranthranilsäure ausgehend von 3,4-Difluoranilin gemäß US 4,833,270 nur 30 % d.Th. Schließlich ist auch bereits die Ammonolyse von Halogenphthalsäureanhydriden zu den Phthalsäureimiden mit nachfolgender Hofmann-Umlagerung und Abbau der Isocyanate zu den Halogenanthranilsäuren bekannt (CA 113 8916 w (1990) und Synthetic communications 15(6) S. 485 - 489 (1985)).

Da die Ringöffnung des Phthalsäureimids nicht selektiv ist und somit außer bei 4,5-disubstituierten Phthalimiden zwei verschiedene Endprodukte entstehen können, ist diese Methode nicht allgemein anwendbar.

Weiterhin ist in der Literatur bereits eine durch Kupfer katalysierte Ammonolyse von Halogenarylen beschrieben. Beispielsweise kann auf diesem Wege 4-Bromchlorbenzol in wäßrigem Ammoniak in 10 Stunden bei 120°C und ca. 10 bar selektiv zu 4-Chloranilin umgesetzt werden (P. H. Groggins: Unit Processes in Organic Synthesis (Mc Graw-Hill Book Co., Inc., New York, NY 1952), Amination by ammonolysis S. 340 - 414, S. 377).

Die EP 51 783 beschreibt die Umsetzung von 1-Amino-4-bromanthrachinon-2-sulfonsäure in 25 % Ammoniak bei 80°C und 15 bar in Gegenwart von katalytischen Mengen CuSO₄, 5H₂O zur entsprechenden 1,4-Diaminoverbindung in einer 10stündigen Reaktionszeit.

Aus der HU 54 104 ist die Ammonolyse von 3,5-Dihydroxy-4-brombenzoesäure bzw. von 3,5-Dialkoxy-4-brombenzoesäurealkylester in 35 %iger Ammoniaklösung bei 118 -128°C in 8 - 11 Stunden in Gegenwart von CuSO₄ oder CuO zu den entsprechenden 4-Aminoverbindungen bekannt.

Ebenfalls unter Kupferkatalyse wird bei einem in der deutschen Patentschrift Nr. 244 207 beschriebenen Verfahren zur Herstellung von Chloranthranilsäuren gearbeitet, bei dem Polychlorbenzoesäuren, bei denen sich mindestens ein Chloratom in o-Stellung zur Carboxylgruppe gefindet, mit Ammoniak bei Temperaturen zwischen 100 und 150°C umgesetzt werden.

In der EP-A-370 686 wird die Umsetzung von chlor- und/oder fluorsubstituierten Benzoesäuren, die in o-Stellung zur Säuregruppe ein Iod-, Brom- oder Chloratom tragen, mit primären Aminen unter Kupferkatalyse und in Gegenwart eines Pyridins in dipolaren aprotischen Lösungsmitteln offenbart.

Die vorliegende Erfindung betrifft ein Verfahren zur Herstellung von Verbindungen der allgemeinen Formel I, 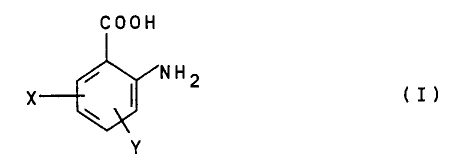
worin X und Y unabhängig voneinander Wasserstoff, Fluor, Chlor oder Brom bedeuten, aber nicht gleichzeitig für Wasserstoff stehen können und wobei die 6-Stellung nicht durch Brom substituiert sein kann, dadurch gekennzeichnet, daß Verbindungen der allgemeinen Formel II 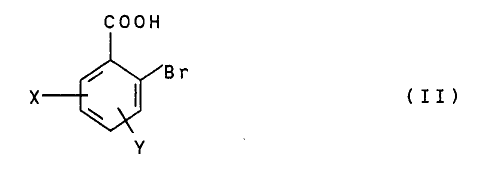
worin X und Y wie oben angegeben definiert sind, mit wäßrigem Ammoniak drucklos bei Temperaturen von 20 bis 40°C unter Kupfer-Katalyse umgesetzt werden.

In den allgemeinen Formeln I und II können sich die Substituenten X und Y in beliebigen Positionen des Benzolringes befinden.

Bevorzugte Verbindungen der allgemeinen Formel I sind 4-Fluoranthranilsäure, 4-Bromanthranilsäure, 5-Chloranthranilsäure, 4-Brom-5-chloranthranilsäure, sowie 4,5-Difluoranthranilsäure.

Unter wäßrigem Ammoniak werden bevorzugt 20 bis 40 %ige Ammoniaklösungen verstanden. Besonders bevorzugt sind die handelsüblichen 25- bzw. 33 %igen Lösungen.

Bevorzugt wird der Ammoniak in einem Überschuß von 1 : 3 bis 1 : 6 über der stöchiometrisch erforderlichen Menge von 3 Moläquivalenten, bezogen auf die Verbindung der allgemeinen Formel II; eingesetzt.

Unter Kupfer-Katalyse wird bevorzugt der Einsatz einer Kupfer(I)verbindung, besonders bevorzugt von Kupfer(I)oxid, verstanden. Der Kupferkatalysator wird dabei bevorzugt in einem molaren Verhältnis von 0,09 : 1 bis 0,15 : 1, bezogen auf die Verbindung der allgemeinen Formel II, eingesetzt.

Vorteilhafterweise wird die erfindungsgemäße Umsetzung in einem inerten Lösungsmittel ausgeführt. Die Wahl eines geeigneten inerten Lösungsmittels richtet sich dabei in erster Linie nach praktischen Gesichtspunkten, wie beispielsweise Löslichkeit der Edukte. Geeignete inerte Lösungsmittel sind beispielsweise Essigester und Isopropanol.

Die Reaktion ist nach etwa 40 bis 180 Minuten beendet.

Es ist besonders bevorzugt am Ende der Reaktion und vor Aufarbeitung des Reaktionsansatzes das eingesetzte Kupfer mit Hilfe eines geeigneten chelatisierenden Agenz zu komplexieren. Ein geeignetes chelatisierendes Agenz ist insbesondere Ethylendiamintetraessigsäure (EDTA).

Da bei dem erfindungsgemäßen Verfahren überraschenderweise nur das ortho-ständige Bromatom ammonolysiert wird und Halogensubstituenten einschließlich Brom an anderen Ringpositionen unverändert bleiben, können die Verbindungen der allgemeinen Formel II auch in Mischungen mit zum Beispiel isomeren Verbindungen, wie sie unter Umständen bei ihrer Synthese anfallen, eingesetzt werden. Beispielsweise wird in einer Mischung aus 2-Brom-4-fluorbenzoesäure und 4-Brom-2-fluorbenzoesäure nur ersteres umgesetzt, letzteres kann anschließend leicht von der gewünschten Anthranilsäure abgetrennt werden.

Die Verbindungen der allgemeinen Formel II sind teilweise bekannt und können nach bekannten Methoden hergestellt werden. Beispielsweise kann 2-Brom-4-fluorbenzoesäure aus 1-Brom-3-fluorbenzol durch Acetylierung und Oxidation des entstandenen 2-Brom-4-fluoracetophenons gewonnen werden (Recl. Trav. Chim. Pays-Bas, 83 S. 1142, 1146 (1964)). In J. Karnatak Univ. 1 S. 36 (1956) ist die Oxidation von 2,4-Dibromacetophenonen mit Kaliumpermanganat zu 2,4-Dibrombenzoesäure beschrieben. Gemäß J. Chem. Soc. 85, 1267 f. (1904) kann 5-Chlor-2-brombenzoesäure durch Salpetersäure-Oxidation von 5-Chlor-2-bromtoluol erhalten werden.

Dagegen sind Vorstufen der allgemeinen Formel III 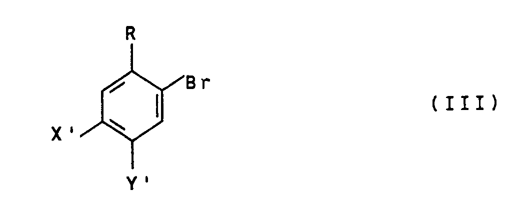
worin
R CH₃ und X' und Y' unabhängig voneinander Fluor oder Chlor bedeuten oder X' für Chlor und Y' für Brom stehen oder worin R COOH und X' und Y' Fluor, Chlor oder Brom bedeuten aber nicht identisch sein können, noch neu und ebenfalls Gegenstand vorliegender Erfindung.

Bevorzugt bedeuten R CH₃ oder COOH und X' Chlor und Y' Brom.

Verbindungen der allgemeinen Formel III, in denen R für COOH steht, fallen unter die allgemeine Formel II und sind direkte Vorstufen der Verbindungen der allgemeinen Formel I gemäß erfindungsgemäßem Verfahren.

Verbindungen der allgemeinen Formel III, in denen R für CH₃ steht, können durch Oxidation der Methylgruppe in Verbindungen der allgemeinen Formel III, in denen R für COOH steht, überführt werden.

Die Verbindungen der allgemeinen Formel III, in denen R für CH₃ steht, können nach den allgemein bekannten Methoden der Halogenaromatenchemie hergestellt werden.

Somit kann beispielsweise 4-Brom-5-chloranthranilsäure nach folgendem Schema hergestellt werden: 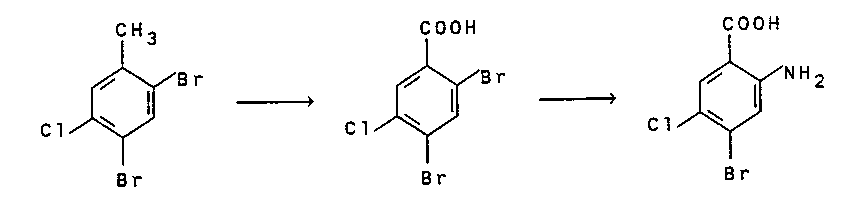
Dabei kann die Ausgangsverbindung 5-Chlor-2-4-dibromtoluol beispielsweise durch Bromierung von 3-Chlortoluol oder von 2-Brom-5-chlortoluol erhalten werden.

### Beispiele:

### 1. Synthese von 4-Fluoranthranilsäure

In einem Glaskolben legt man 1,3 g Kupfer(I)oxid (0,009 mol) in 69 g (1 mol NH₃) einer wäßrigen 25 %igen Ammoniaklösung unter Stickstoff vor. Dazu gibt man unter Rühren bei 25°C innerhalb von 5 min eine Lösung von 21,9 g (0,1 mol) 2-Brom-4-fluorbenzoesäure in 18 g (0,27 mol) 25 %ige Ammoniaklösung und 60 ml Essigsäureethylester zu. Die Reaktion wird bis zum Ende unter Stickstoff durchgeführt. Nach Zugabe der Ammoniumbenzoatlösung steigt die Reaktionstemperatur bis auf ca. 40°C. Die anfangs rötliche Kupfer(I)oxidsuspension geht in eine tiefblaue Lösung über. Nach 1 h Rühren bei Raumtemperatur ist die Reaktion beendet. Zum Komplexieren des Kupfers gibt man 5,3 g Ethylendiamintetraessigsäure in den Ansatz, säuert mit Salzsäure auf pH 3,1 an und destilliert den Essigester ab. Das Produkt wird bei Raumtemperatur isoliert.
Ausbeute:
14,6 g 4-Fluoranthranilsäure, das entspricht 94 % d.Th.
HPLC: 99 %
Fp.: 192 - 194°C
¹³C-NMR (75 MHz, ¹H-breitbandentkoppelt, DMSO-d₆) δppm
101,225 (d,²J(C-CF) = 24,3 Hz, arom. C)
102,38 (d,²J(C-CF) = 23,2 Hz, arom. C)
106,825 (arom. C)
134,18 (d,³J(C-CF) = 12 Hz, arom. C)
153,74 (d,³J(C-CF) = 13,36 Hz, arom. C)
165,75 (d,¹J(C-CF) = 247 Hz, arom. C)
168,82 (-COOH)

### 2. Synthese von 4-Bromanthranilsäure

Man legt unter Stickstoff 4,1 g (0,0287 mol) Kupfer(I)oxid in 143 g (2,1 mol NH₃) einer wäßrigen 25 %igen Ammoniaklösung vor. Dazu tropft man unter Rühren innerhalb von 30 min ein zweiphasiges Gemisch, bestehend aus 58 g (0,207 mol) 2,4-Dibrombenzoesäure in 56 g (0,8 mol NH₃) Ammoniaklösung und 130 ml Essigsäureethylester zu. Die Temperatur steigt während der Dosierung von 18 - 40°C an. Man rührt 15 min bei 40°C nach. Kupfer wird mit EDTE komplexiert. Die Anthranilsäure wird durch Ansäuern mit Salzsäure bis pH 3,1 gefällt und nach üblicher Laboratoriumspraxis isoliert.
Ausbeute:
40,9 g (0,189 mol) 4-Bromanthranilsäure, das entspricht
91,5 % d.Th.
HPLC: 97,3 %
300 MHz ¹H-NMR (DMSO-d₆)
- δ (ppm):: 6,65 (dd, J = 9 Hz, J = 2 Hz, 1H)
6,98 (d, J = 2 Hz, 1H)
7,6 (d, J = 9 Hz, 1H)

### 3. Synthese von 4-Brom-5-chloranthranilsäure

a) Zu 316,5 g (2,5 mol) 3-Chlortoluol und 12,5 g getrocknetem Eisen(III)chlorid in 950 ml 1-Brombutan tropft man bei 0°C unter Rühren 799 g (5 mol) Brom. Es wird 4 h dosiert und ca. 1/2 h bei 0°C nachgerührt. Nach Abklingen der Bromwasserstoffentwicklung versetzt man mit 250 ml Wasser. Nach Phasentrennung wird die organische Phase mit 250 ml gesättigter Natriumhydrogencarbonatlösung gewaschen. Von dem erhaltenen Rohprodukt wird das 1-Brombutan abdestilliert. Man erhält 710 g (2,5 mol) 5-Chlor-2,4-dibromtoluol mit einer GC-Gehalt von 69,5 % und einem Isomerenanteil von 18 %.
   Schmelzbereich 70 - 75°C.
   Durch Umkristallisation des Rohproduktes aus unpolaren und dipolar aprotischen Lösungsmitteln, wie z.B. Benzin, Petrolether, Dichlormethan, 1,2-Dichlorethan, iso-Propylbromid, 1-Brombutan, erhält man 5-Chlor-2,4-dibromtoluol in einer Ausbeute von 79 % d.Th., bezogen auf 3-Chlortoluol. Die mittlere Reinheit beträgt 97 - 99,8 %, der Schmelzbereich 92 - 96°C.
   ¹H-NMR (300 MHz in DMSO-d₆) δ-Werte in ppm bezogen auf TMS.
   2,30 (3 H, s)
   7,79 (1 H, s)
   7,87 (1 H, s)
b) In einem Rundkolben werden 51,4 g (0,25 mol) 2-Brom-5-chlortoluol, 25 ml Dichlorethan und 1,5 g Eisenpulver vorgelegt. Unter Rühren werden bei 38 - 42°C 40 g (0,25 mol) Brom eingetropft, wobei Bromwasserstoff freigesetzt wird. Während der Dosierung fällt das Produkt aus. Man hält die Suspension durch Zugabe von weiteren 35 ml Dichlormethan gut rührfähig. Nach Zugabe des Broms läßt man 30 min nachrühren. Bei Raumtemperatur wird der Kristallbrei mit 20 ml Dichlormethan in Lösung gebracht und das Eisenpulver abfiltriert. Die organische Phase wird am Rotavapor eingeengt. Man erhält 58 g 5-Chlor-2,4-dibromtoluol, das entspricht 81,6 % d.Th., bezogen auf 2-Brom-5-chlortoluol, Gehalt (aus GC): 91 %, Schmelzbereich 91 - 95°C.
c) 5-Chlor-2,4-dibrombenzoesäure
   In einer Hastelloy-C Druckapparatur werden 299 g (1,05 mol) 5-Chlor-2,4-dibromtoluol (Reinheit lt. GC 97 %) in 3000 g Essigsäure unter Katalyse von 130,8 g Kobalt(II)acetat·4H₂O, 4,4 g Mangan(II)acetat·4H₂O und 35 g Natriumbromid bei einem Druck von 5 bar und ca. 130°C Reaktionstemperatur innerhalb von 6 h mit Preßluft zur 5-Chlor-2,4-dibrombenzoesäure oxidiert. Nach beendeter Reaktion destilliert man unter Normaldruck 2 l Essigsäure ab und fällt die 5-Chlor-2,4-dibrombenzoesäure mit 4 l Wasser aus. Das Rohprodukt wird anschließend in 1,5 l Wasser und 150 ml 32 %iger Natronlauge zur Lösung auf 70°C erwärmt, gekohlt und im Filtrat das Produkt mit 180 l 32 %iger Salzsäure bis pH 1 ausgefällt. Man erhält nach Absaugen, Waschen und Trocknen 247,7 g 5-Chlor-2,4-dibrombenzoesäure, entsprechend 75 % d.Th.
   HPLC: 99,8 %
   ¹H-NMR (300 MHz, DMSO-d₆) δ-Werte in ppm
   8,05 (1 H, s); 8,08 (1 H, s)
   12,0 - 15,7 (1 H, breit H/D)
   ¹³C-NMR (75 MHz, ¹H-breitbandentkoppelt, DMSO-d₆) δ ppm
   119,14 (arom. C)
   125,218 (arom. C)
   131,49 (arom. C)
   132,9175 (arom. C)
   134,36 (arom. C)
   137,98 (arom. C)
   165,50 (-COOH)
d) 4-Brom-5-chloranthranilsäure
   2,8 g (0,0196 mol) Kupfer(I)oxid suspendiert man in 120 ml (entsprechend 1,6 mol NH₃) 25 % wäßriger Ammoniaklösung unter Stickstoff. Dazu tropft man bei 25°C unter Rühren innerhalb von 40 min eine Suspension von 63,2 g (0,2 mol) 5-Chlor-2,4-dibrombenzoesäure, 160 ml (entsprechend 2,14 mol NH₃) 25 % NH₃·aqu. und 180 ml Essigester unter Sauerstoffausschluß zu. Die Temperatur steigt während der Dosierung auf ca. 35°C an. Man läßt 2 1/4 h bei 30°C nachrühren. Anschließend gibt man 11,6 g (0,04 mol) Ethylendiamintetraessigsäure in den Ansatz und säuert mit Salzsäure bis pH 3,1 an. Der Essigester wird bis 90°C abdestilliert und die Anthranilsäure nach Kaltrühren der Suspension abgesaugt und gewaschen. Man erhält 48,2 g 4-Brom-5-chloranthranilsäure als beiges Pulver, das entspricht 96,2 % d.Th., bez. auf 5-Chlor-2,4-dibrombenzoesäure.
   Gehalt (HPLC): 95 %
   ¹³C-NMR(75 MHz, ¹H-breitbandentkoppelt in DMSO-d₆)
   δ-Werte in ppm arom. = aromatisch
   110,494 (arom. C)
   117,423 (arom. C)
   120,652 (arom. C)
   126,907 (arom. C)
   131,726 (arom. C)
   150,743 (arom. C)
   167,913 (-COOH)

### 4. Synthese von 5-Chloranthranilsäure

Es wird analog Beispiel 1 gearbeitet.
Ausbeute: 97 % d.Th.
HPLC: 90 %
Fp.: 233 - 235°C

### 5. Synthese von 4,5-Difluoranthranilsäure

Es wird analog Beispiel 1 gearbeitet.
Ausbeute: 91 % d.Th.
HPLC: 99 %
Fp.: 180 - 182°C

## Patentansprüche

1. Verfahren zur Herstellung von Verbindungen der allgemeinen Formel I 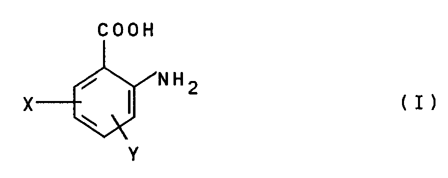 worin X und Y unabhängig voneinander Wasserstoff, Fluor, Chlor oder Brom bedeuten, aber nicht gleichzeitig für Wasserstoff stehen können und wobei die 6-Stellung nicht durch Brom substituiert sein kann, dadurch gekennzeichnet, daß Verbindungen der allgemeinen Formel II 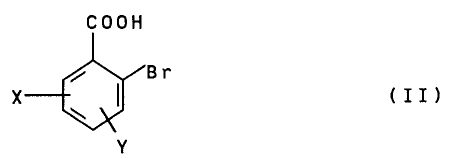 worin X und Y wie oben angegeben definiert sind, mit wäßrigem Ammoniak drucklos bei Temperaturen von 20 bis 40°C unter Kupfer-Katalyse umgesetzt werden.

2. Verfahren gemäß Anspruch 1, dadurch gekennzeichnet, daß als wäßriger Ammoniak 20- bis 40 %ige, besonders bevorzugt 25 - 33 %ige, Ammoniaklösungen eingesetzt werden.

3. Verfahren gemäß Anspruch 1 und/oder 2, dadurch gekennzeichnet, daß Ammoniak in einem Überschuß von 1 : 3 bis 1 : 6 über der stöchiometrisch erforderlichen Menge von 3 Moläquivalenten, bezogen auf die Verbindung der allgemeinen Formel II eingesetzt wird.

4. Verfahren gemäß einem oder mehreren der Ansprüche 1 bis 3, dadurch gekennzeichnet, daß als Kupferkatalysator eine Kupfer(I)verbindung, besonders bevorzugt Kupfer(I)oxid, eingesetzt wird.

5. Verfahren gemäß einem oder mehreren der Ansprüche 1 bis 4, dadurch gekennzeichnet, daß der Kupferkatalysator in einem molaren Verhältnis von 0,09 : 1 bis 0,15 : 1, bezogen auf die Verbindung der allgemeinen Formel II eingesetzt wird.

6. Verfahren gemäß einem oder mehreren der Ansprüche 1 bis 5, dadurch gekennzeichnet, daß die Umsetzung in einem inerten Lösungsmittel ausgeführt wird.

7. Verfahren gemäß einem oder mehreren der Ansprüche 1 bis 6, dadurch gekennzeichnet, daß vor Aufarbeitung des Reaktionsansatzes das eingesetzte Kupfer mit Hilfe eines geeigneten chelatisierenden Agenz komplexiert wird.

8. Verbindung der allgemeinen Formel III 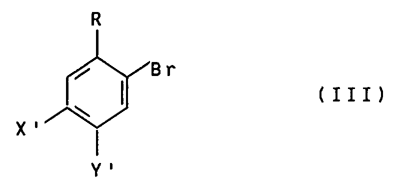 worin R CH₃ und X' und Y' beide Chlor, beide Fluor oder X' Chlor und Y' Fluor oder Brom bedeuten oder worin R COOH und X' Chlor oder Brom und Y' Fluor, Chlor oder Brom bedeuten, aber X' und Y' nicht identisch sein können.

9. Verbindung gemäß Anspruch 8, dadurch gekennzeichnet, daß R COOH, X' Chlor und Y' Brom bedeuten.

## Claims

1. Process for the preparation of compounds of the general formula I 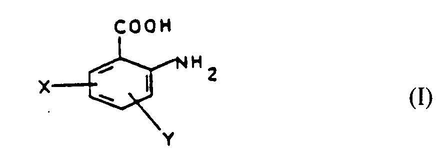 in which X and Y, independently of each other, represent hydrogen, fluorine, chlorine or bromine, but cannot stimultaneously represent hydrogen, and wherein the 6-position may not be substituted by bromine, characterized in that compounds of the general formula II 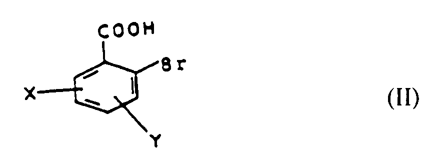 in which X and Y are defined as above are reacted with aqueous ammonia by copper catalysis without pressure at temperatures of from 20° to 40° C.

2. Process according to claim 1 characterized in that the aqueous ammonia used is a 20 to 40% strength, preferrably 25 to 33 % strength ammonia solution.

3. Process according to claim 1 and/or 2 characterized in that ammonia is used in an excess of 1:3 to 1:6 above the stoichiometrically required amount of three molar equivalents, relative to the compound of the general formula II.

4. Process according to one or more of claims 1 to 3, characterized in that the copper catalyst used is a copper (I) compound, particularly preferrably copper (I) oxide.

5. Process according to one or more of claims 1 to 4, characterized in that the copper catalyst is used in a molar ratio of 0.09:1 to 0.15:1, relative to the compound of the general formula II.

6. Process according to one or more of the claims 1 to 5 characterized in that the reaction is carried out in an inert solvent.

7. Process according to one or more the claims 1 to 6, characterized in that the used copper is complexated by means of a suitable chelating agent prior to the work-up of the reaction mixture.

8. Compound of the general formula III 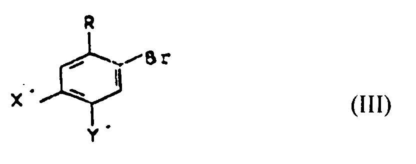 in which R represents CH₃ and X' and Y' represent both chlorine or both fluorine or X' represents chlorine and Y' represents fluorine or bromine or in which R represents COOH and X' represents chlorine or bromine and Y' represents fluorine, chlorine or bromine, but X' and Y' cannot be identical.

9. Compound according to claim 8, characterized in that R represents COOH, X' represents chlorine and Y' represents bromine.

## Revendications

1. Procédé pour préparer des composés ayant la formule générale I 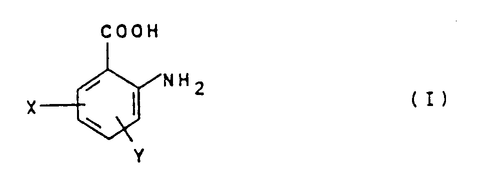 dans laquelle X et Y, indépendamment l'un de l'autre, sont chacun un hydrogène, un fluor, un chlore ou un brome, mais ne peuvent être simultanément des hydrogènes, et la position 6 ne pouvant être substituée par le brome, caractérisé en ce qu'on fait réagir des composés de formule générale II 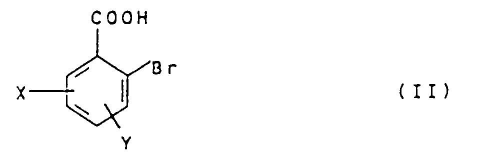 dans laquelle X et Y ont les significations données ci-dessus, avec de l'ammoniaque, sans pression, à des températures de 20 à 40°C, la réaction étant catalysée par le cuivre.

2. Procédé selon la revendication 1, caractérisé en ce qu'on utilise comme ammoniaque des solutions ammoniaquées à 20-40 %, et d'une manière particulièrement préférée à 25-33 %.

3. Procédé selon la revendication 1 et/ou 2, caractérisé en ce que l'ammoniaque est utilisé selon un excès de 1:3 à 1:6 par rapport à la quantité nécessaire d'un point de vue stoechiométrique de 3 équivalents en mole, par rapport au composé de formule générale II.

4. Procédé selon l'une ou plusieurs des revendications 1 à 3, caractérisé en ce qu'on utilise comme catalyseur au cuivre un composé du cuivre(I), d'une manière particulièrement préférée l'oxyde de cuivre(I).

5. Procédé selon l'une ou plusieurs revendications 1 à 4, caractérisé en ce que le catalyseur au cuivre est utilisé selon un rapport en moles de 0,09:1 à 0,15:1 par rapport au composé de formule générale II.

6. Procédé selon l'une ou plusieurs des revendications 1 à 5, caractérisé en ce que la réaction a lieu dans un solvant inerte.

7. Procédé selon l'une ou plusieurs des revendications 1 à 6, caractérisé en ce que, avant traitement de la masse réactionnelle, le cuivre utilisé est complexé à l'aide d'un agent chélatant approprié.

8. Composé de formule générale III 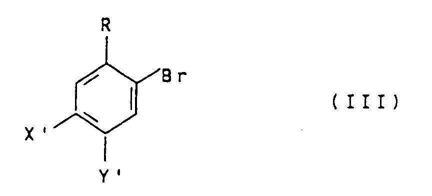 dans laquelle R est CH₃, et X' et Y' sont tous les deux le chlore, tous les deux sont le fluor, ou encore X' est le chlore et Y' est le fluor ou le brome, ou encore dans laquelle R est COOH et X' est le chlore ou le brome et Y' est le fluor, le chlore ou le brome, mais X' et Y' ne peuvent être identiques.

9. Composé selon la revendication 8, caractérisé en ce que R est COOH, X' est le chlore et Y' est le brome.
